# EUROPÄISCHE PATENTSCHRIFT

(11) **EP 3 153 151 B1**
(45) Veröffentlichungstag und Bekanntmachung des Hinweises auf die Patenterteilung: **11.08.2021**
(21) Anmeldenummer: 16186938.3
(22) Anmeldetag: 02.09.2016
(51) Int. Cl.: A61K 8/362, A61K 8/49, A61K 8/55, A61K 8/73, A61Q 17/04

(54) **SONNENSCHUTZMITTEL MIT STARK REDUZIERTER TEXTILVERFLECKUNG DURCH 4-(TERT.-BUTYL)-4 - METHOXYDIBENZOYLMETHAN**
SUNSCREEN HAVING REDUCED TENDENCY TO CAUSE 4-(TERT.-BUTYL)-4 - METHOXYDIBENZOYLMETHAN STAINS ON TEXTILES
PRODUIT DE PROTECTION SOLAIRE AYANT UNE TENDANCE RÉDUITE À FORMER DES TACHES CAUSÉ PAR 4-(TERT.-BUTYL)-4 - METHOXYDIBENZOYLMETHANSUR LES TEXTILES

(30) Priorität: 09.10.2015 DE 102015219591
(43) Veröffentlichungstag der Anmeldung: 12.04.2017
(73) Patentinhaber: Beiersdorf AG, 20253 Hamburg (DE)
(72) Erfinder: Weinert, Katrin, 22763 Hamburg (DE); Borchers, Kathrin, 21614 Buxtehude (DE); Bleckmann, Andreas, 22926 Ahrensburg (DE)

(56) Entgegenhaltungen:
- EP-A1- 1 834 631
- EP-A1- 3 150 189
- WO-A1-2014/028032
- WO-A1-2015/165715
- WO-A1-2016/062543
- WO-A2-2014/012699
- DE-A1-102010 008 320
- "Face cream SPF 50", GNPD, April 2015 (2015-04), XP002764290,
- "Face cream SPF 50", GNPD, July 2015 (2015-07), XP002764291,

## Beschreibung

Die vorliegende Erfindung betrifft eine kosmetische Zubereitung enthaltend 4-(tert.-Butyl)-4'-methoxydibenzoylmethan und mindestens zwei Additive gewählt aus der Gruppe der Verbindungen Cellulose Gum, Iminodisuccinat, 1-Hydroxy-4-methyl-6-(2,4,4-trimethylpentyl)-2(1H)-pyridon (Pirocton) und Diethylentriaminpenta(methylenphosphonsäure)/ DTPMP.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurde daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter wie 4-(tert.-Butyl)-4'-methoxydibenzoylmethan hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch.

Es war daher die Aufgabe der vorliegenden Erfindung, die Nachteile des Standes der Technik zu beseitigen und eine kosmetische Zubereitung (insbesondere ein Sonnenschutzmittel) enthaltend nicht-wasserlösliche UV-A Filter wie 4-(tert.-Butyl)-4'-methoxydibenzoylmethan zu entwickeln, welche sich leichter aus den mit der Zubereitung kontaminierten Textilen herauswaschen lassen.

Überraschend gelöst wird die Aufgabe durch eine kosmetische Zubereitung gemäß Anspruch 1.

Zwar kennt der Stand der Technik die DE102004011111, DE 102010054918, DE 10201005465, DE 102010054866, DE 19639817, EP0928193, EP 1088546 und EP0463780, doch konnten diese Schriften nicht den Weg zur vorliegenden Erfindung weisen. Darüber hinaus kennt der Fachmann die DE102010008320, WO 2014/028032, EP 1834631 und WO2014/012699 sowie die nachveröffentlichten WO2016/062543, WO2015/165715, EP3150189 und die in der GNPD (Mintel) Datenbank veröffentlichten zwei Produkte Face Cream SPF 50 (Record ID 3101737 und Record ID 3354913), die ebenfalls nicht den Weg zur vorliegenden Erfindung weisen konnten.

Darüber hinaus gehört zum Stand der Technik nach Artikel 54(3) EPÜ das Dokument WO 2016/062543 A1, dessen Rezepturen 18, 19 und 20 auf den Seiten 14/15 aus dem Schutzbereich der vorliegenden Erfindung mit Hilfe eines Disclaimers ausgeklammert werden.

Erfindungsgemäß besonders bevorzugt enthält die erfindungsgemäße Zubereitung neben 4-(tert.-Butyl)-4'-methoxydibenzoylmethan und Cellulose Gum zwei weitere Additive gewählt aus der Gruppe der Verbindungen Iminodisuccinat, 1-Hydroxy-4-methyl-6-(2,4,4-trimethylpentyl)-2(1H)-pyridon (Pirocton) und Diethylentriaminpenta(methylenphosphonsäure)/ DTPMP.

Es ist erfindungsgemäß vorteilhaft, wenn die Zubereitung 4-(tert.-Butyl)-4'-methoxydibenzoylmethan in einer Menge von 0,01 bis 5 Gewichts-%, bezogen auf das Gesamtgewicht der Zubereitung, enthält. Erfindungsgemäß bevorzugt ist dabei ein Gehalt von 2 bis 5 Gewichts-%, bezogen auf das Gesamtgewicht der Zubereitung.

Das erfindungsgemäß vorteilhafte Cellulose Gum wird auch als Cellulosegummi oder NatriumCarboxymethylcellulose bezeichnet. Erfindungsgemäß vorteilhafte Ausführungsformen der vorliegenden Erfindung sind dadurch gekennzeichnet, dass die Zubereitung Cellulose Gum in einer Konzentration von 0,01 bis 2,0 Gewichts-%, bezogen auf das Gesamtgewicht der Zubereitung, enthält. Erfindungsgemäß bevorzugt sind Einsatzkonzentrationen von 0,1 bis 1,0 Gewichts-%, bezogen auf das Gesamtgewicht der Zubereitung.

Das erfindungsgemäß vorteilhafte Iminosuccinat wird vorteilhafter Weise in Form seines Tetranatrium-Salzes (INCI Tetrasodium Iminodisuccinate) eingesetzt.
Es ist erfindungsgemäß vorteilhaft, wenn die Zubereitung Iminodisuccinat in einer Konzentration von 0,01 bis 2,0 Gewichts-%, bezogen auf das Gesamtgewicht der Zubereitung, enthält. Erfindungsgemäß bevorzugt sind Einsatzkonzentrationen von 0,1 bis 1,0 Gewichts-%, bezogen auf das Gesamtgewicht der Zubereitung.

Die erfindungsgemäß vorteilhafte Diethylentriaminpenta(methylenphosphonsäure), abgekürzt DTPMP, wird erfindungsgemäß bevorzugt in Form ihres Natrium- oder Kaliumsalzes eingesetzt, wobei das Natriumsalz besonders bevorzugt ist.
Es ist vorteilhaft im Sinne der vorliegenden Erfindung, wenn die Zubereitung Diethylentriaminpenta(methylenphosphonsäure)/ DTPMP in einer Konzentration von 0,01 bis 1,0 Gewichts-%, bezogen auf das Gesamtgewicht der Zubereitung, enthält. Erfindungsgemäß bevorzugt sind Einsatzkonzentrationen von 0,05 bis 0,5 Gewichts-%, bezogen auf das Gesamtgewicht der Zubereitung.

Darüber hinaus ist es vorteilhaft im Sinne der vorliegenden Erfindung, wenn die Zubereitung 1-Hydroxy-4-methyl-6-(2,4,4-trimethylpentyl)-2(1H)-pyridon (Pirocton) in einer Gesamtmenge von 0,01 bis 1,0 Gewichts-%, bezogen auf das Gesamtgewicht der Zubereitung, enthält. Erfindungsgemäß bevorzugt ist dabei ein Gehalt von 0,1 bis 0,5 Gewichts-%, bezogen auf das Gesamtgewicht der Zubereitung.

Der Zusatz von Pirocton kann dabei erfindungsgemäß auf zwei Wegen erfolgen. Zum einen kann das Piroton per se der Zubereitung zugesetzt werden. Darüber hinaus kann es aber auch in Form seines Monoethanolamin-Salzes zugesetzt werden. Beide Wege sind erfindungsgemäß.

Die erfindungsgemäß bevorzugte Variante ist jedoch dadurch gekennzeichnet, dass das 1-Hydroxy-4-methyl-6-(2,4,4-trimethylpentyl)-2(1H)-pyridon (Pirocton) in Form seines Monoethanolamin-Salzes (Piroctone Olamine) eingesetzt wird.

Wird das Pirocton in Form von Piroctone Olamine zugefügt, so ist es erfindungsgemäß vorteilhaft, diesen Rohstoff in einer Menge von 0,01 bis 1,0 Gewichts-%, bezogen auf das Gesamtgewicht der Zubereitung und bevorzugt in einer Menge von 0,1 bis 0,5 Gewichts-%, bezogen auf das Gesamtgewicht der Zubereitung, einzusetzen.

Die erfindungsgemäße Zubereitung kann in Form eines alkoholischen Sprays oder Gels oder in Form einer Emulsion vorliegen.

Liegt die Zubereitung in Form eines alkoholischen Sprays oder Gels vor, so ist es erfindungsgemäß vorteilhaft, wenn der Gehalt an Ethanol von 20 bis 70 Gewichts-%, bezogen auf das Gesamtgewicht der Zubereitung beträgt.

Liegt die erfindungsgemäße Zubereitung in Form einer Emulsion vor, so ist es erfindungsgemäß vorteilhaft, wenn es sich dabei um eine O/W-Emulsion handelt.

Darüber hinaus sind derartige Emulsionen als erfindungsgemäß vorteilhaft dadurch gekennzeichnet, dass die Zubereitung einen oder mehrere Emulgatoren gewählt aus der Gruppe der Verbindungen Glycerylstearatcitrat, Cetearylalkohol, Natriumcetearylsulfat, Glycerylstearat, Cetearylsulfosuccinat, Natriumstearoylglutamat, Polyglyceryl-3-methylglucosedistearat, Sucrose Polystearate, Stearinsäure, Polyglyceryl-10 Stearat, Kaliumcetylphosphat, enthält.

Es ist vorteilhaft im Sinne der vorliegenden Erfindung, wenn die kosmetische Zubereitung dadurch gekennzeichnet ist, dass die Zubereitung einen oder mehrere UV-Filter enthält, die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)-benzolsulfonsäuresalze; 2-Methyl-5-(2-oxo-3-bornyl idenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat;
Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Erfindungsgemäß bevorzugt ist es dabei, wenn die erfindungsgemäße Zusammensetzung 2-(4'-Diethylamino-2'-hydoxybenzoyl)-benzoesäurehexylester und/oder 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin), enthält.

Die erfindungsgemäß vorteilhafte Einsatzkonzentration für 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) beträgt dabei von 0,1 bis 10 Gewichts-%, bezogen auf das Gesamtgewicht der Zubereitung.

Die erfindungsgemäß bevorzugte Einsatzkonzentration für 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) beträgt dabei von 0,5 bis 5 Gewichts-%, bezogen auf das Gesamtgewicht der Zubereitung.

Die erfindungsgemäß vorteilhafte Einsatzkonzentration für 2-(4'-Diethylamino-2'-hydoxybenzoyl)-benzoesäurehexylester beträgt dabei von 0,1 bis 10 Gewichts-%, bezogen auf das Gesamtgewicht der Zubereitung.

Die erfindungsgemäß bevorzugte Einsatzkonzentration für 2-(4'-Diethylamino-2'-hydoxybenzoyl)-benzoesäurehexylester beträgt dabei von 0,5 bis 5 Gewichts-%, bezogen auf das Gesamtgewicht der Zubereitung.

Es ist erfindungsgemäß vorteilhaft, wenn die Zubereitung eine oder mehrere Verbindungen gewählt aus der Gruppe der Verbindungen Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

Darüber hinaus ist es erfindungsgemäß von Vorteil, wenn die Zubereitung Phenoxyethanol und/oder Methylparaben enthält.

Dabei ist es erfindungsgemäß bevorzugt, wenn die Zubereitung Phenoxyethanol in einer Konzentration von 0,01 bis 1 Gewichts-%, bezogen auf das Gesamtgewicht der Zubereitung, enthält.

Es ist erfindungsgemäß von Vorteil, wenn die Zubereitung einen oder mehrere Wirkstoffe gewählt aus der Gruppe der Verbindungen Magnolienextrakt, alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, β-Alanin, Tocopherylacetat, Panthenol, Magnolol, Honokiol, Harnstoff, Hyaluronsäure, Dihydroxyaceton, 8-Hexadecen-1,16-dicarbonsäure, Glycyrrhetinsäure, Glucosylglyceride und/oder Licochalcon A enthält.

Erfindungsgemäß besonders bevorzugt ist dabei der Einsatz von Glycyrrhetinsäure bzw. Glycyrrhetinsäure enthaltender Pflanzenextrakte (z.B. *Glycyrrhiza glabra*).

Erfindungsgemäß bevorzugt ist es, wenn die Zubereitung frei ist von Propyl- und Butylparaben, 3-lod-2-propinylbutylcarbamat, 3-(4-Methylbenzyliden)-campher und 2-Hydroxy-4-methoxybenzophenon (Oxybenzon).

Vorteilhaft enthält die erfindungsgemäße Zubereitung Glycerin. Die erfindungsgemäß vorteilhafte Einsatzkonzentration für Glycerin beträgt in den erfindungsgemäßen Zubereitungen von 0,01 bis 15 Gewichts-%, bezogen auf das Gesamtgewicht der Zubereitung.

Erfindungsgemäß vorteilhafte Ausführungsformen erhält man nicht zuletzt dadurch, dass die Zubereitung C12-15 Alkylbenzoat, Cyclomethicon, Octyldodecanol, Capryl/Caprinsäure Triglyceride, Butylenglykol Dicaprylat/Dicaprat, Phenethylbenzoat, Cocoglyceride, Di-n-butyladipat und/oder Diisopropyladipat enthält.

Erfindungsgemäß vorteilhafte Ausführungsformen der vorliegenden Erfindung können vorteilhafter Weise zusätzlich einen oder mehrere weitere Komplexbildner enthalten. Diese können vorteilhaft gewählt werden aus der Gruppe der Verbindungen der EDTA, Aminopolycarboxylate, Polyphosphonate, Polyphosphate und komplexbildenden Säuren.

Dabei ist es erfindungsgemäß bevorzugt, wenn als Komplexbildner eine oder mehrere der Verbindungen aus der Gruppe
- Ethylendiamintetraessigsäure/ EDTA
- 1-Hydroxyethan-(1,1-diphosphonsäure)/ HEDP
- Aminotrimethylenphosphonsäure/ ATMP
- Ethylendiamintetra(methylenphosphonsäure/ EDTMP
- Phosphonobutan-tricarbonsäure/ PBTC
- Natriumpolyphosphat
- Tetranatriumpyrophosphat
- Hydroxamsäure
- Polygalacturonsäure
- Bernsteinsäure
- Ameisensäure
- Äpfelsäure
und/oder deren Alkalisalze eingesetzt werden.

Erfindungsgemäß besonders bevorzugte Ausführungsformen der vorliegenden Erfindung sind dadurch gekennzeichnet, dass als Komplexbildner eine oder mehrere der Verbindungen aus der Gruppe
- Ethylendiamintetraessigsäure/ EDTA
- Ethylendiamintetra(methylenphosphonsäure/ EDTMP
- Aminotrimethylenphosphonsäure/ ATMP
- Natriumpolyphosphat
- Tetranatriumpyrophosphat
- Bernsteinsäure
und/oder deren Alkalisalze eingesetzt werden.

Als erfindungsgemäß vorteilhafte Alkalisalze gelten dabei Natrium- und Kaliumsalze, wobei die Natriumsalze erfindungsgemäß bevorzugt werden.

Es ist vorteilhaft im Sinne der vorliegenden Erfindung, wenn die erfindungsgemäße Zubereitung einen oder mehrere dieser zusätzlichen Komplexbildner in einer Gesamtmenge von 0,1 bis 3 Gewichts-%, bezogen auf das Gesamtgewicht der Zubereitung, enthält.

Es ist bevorzugt im Sinne der vorliegenden Erfindung, wenn die erfindungsgemäße Zubereitung einen oder mehrere dieser zusätzlichen Komplexbildner in einer Gesamtmenge von 0,5 bis 1,5 Gewichts-%, bezogen auf das Gesamtgewicht der Zubereitung, enthält.

Darüber hinaus ist es vorteilhaft im Sinne der vorliegenden Erfindung, wenn die Zubereitung eine oder mehrere Verbindungen aus der Gruppe der Polysaccharide (außer Cellulose Gum) enthält. Diese können erfindungsgemäß vorteilhaft gewählt werden aus der Gruppe der Verbindungen Welan Gum, Sclerotium Gum und Alginaten (insbesondere Sodium Alginate).

Es ist in jedem Falle erfindungsgemäß vorteilhaft, wenn die Zubereitung Polysaccharide in einer Gesamtmenge von 0,01 bis 2 Gewichts-%, bezogen auf das Gesamtgewicht der Zubereitung, enthält.

Es ist vorteilhaft im Sinne der vorliegenden Erfindung, wenn die Zubereitung ein oder mehrere Verdickungsmittel enthält.

Erfindungsgemäß bevorzugt werden dabei ein oder mehrere Verdickungsmittel gewählt aus der Gruppe der Verbindungen Ammonium Acryloyldimethyltaurate/VP Copolymer, Ammonium Acryloyldimethyltaurate/ Beheneth-25 Methacrylate Copolymer, Hydroxypropylcellulose Vinylpyrrolidon/Acrylsäure-Copolymer enthält.

Es ist insbesondere von Vorteil, die Filmbildner aus der Gruppe der Polymere auf Basis von Polyvinylpyrrolidon (PVP) 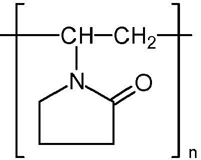 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind. Erfindungsgemäß besonders bevorzugt ist auch das Vinylpyrrolidon/Triaconten-Copolymer Antaron WP-660.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP/VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Es ist ferner vorteilhaft im Sinne der vorliegenden Erfindung, wenn die erfindungsgemäße Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Ethylenbrassylat, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

Erfindungsgemäß vorteilhaft beträgt pH-Wert der erfindungsgemäßen Zubereitung 5-8.

Die erfindungsgemäße Zubereitung kann besonders bevorzugt als Tagespflegeprodukt oder Sonnenschutzmittel eingesetzt werden.

Erfindungsgemäß ist darüber hinaus ein Verfahren zur Erleichterung der Auswaschbarkeit von 4-(tert.-Butyl)-4'-methoxydibenzoylmethan enthaltenden kosmetischen Zubereitungen aus Textilien, gemäß Anspruch 15.

Erfindungsgemäß ist nicht zuletzt die Verwendung nach Anspruch 16 von Cellulose Gum und mindestens einem Additiv gewählt aus der Gruppe der Verbindungen Iminodisuccinat, 1-Hydroxy-4-methyl-6-(2,4,4-trimethylpentyl)-2(1H)-pyridon (Pirocton) und Diethylentriaminpenta(methylenphosphonsäure)/ DTPMP in 4-(tert.-Butyl)-4'-methoxydibenzoylmethan enthaltenden kosmetischen Zubereitungen zur Erleichterung der Auswaschbarkeit der UV-Lichtschutzfilter aus mit den Zubereitungen kontaminierten Textilien. Dabei kann auch hier das Kosmetikum wie oben beschrieben zusammengesetzt sein.

### Vergleichsversuch

Mit dem folgenden Versuch konnte der erfindungsgemäße Effekt beispielhaft belegt werden: Es wurden 0,5% des erfindungsgemäßen Hilfsstoffs Cellulose Gum zu einer Butyl Methoxydibenzoylmethane-enthaltenden Formulierung zugesetzt und die verfleckungsreduzierende Wirkung (Reduktion db) im Vergleich zu einer Formulierung ohne den erfindungsgemäßen Hilfstoff mittels einer *in vitro* Waschmethode bestimmt. Anschließend wurde durch Zugabe der weitern erfindungsgemäßen Hilfsstoffe die synergistische Wirkung von Zweier- und Dreier-Kombinationen bestätigt.

Als Beleg der verbesserten Auswaschbarkeit und verminderten Fleckenbildung der erfindungsgemäßen Zubereitungen wurden in vitro Untersuchungen durchgeführt, deren Ergebnisse in Tabelle 1 dargestellt sind.

Es wurden verschiedene Sonnenschutzemulsionen hinsichtlich der Bildung von gelben Flecken über einen in vitro Auftragungs-/ Waschzyklus untersucht. Es wurden dabei weiße vorgewaschene Baumwollmonitore (100% Baumwolle) verwendet. Dazu wurden je 50 mg der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät spectro-color (Dr. Lange); Farbmess-Software: spectral-QC, Version Messgeometrie: d/8°, Glanzkomponente ausgeschlossen, Lichtart: D65 (entsprechend mittlerem Tageslicht), Kalibrierstandard: LZM 268, Messöffnung: 10mm, Probenhintergrund: Unterlagepapier ohne optischen Aufheller, Prüfklima: 21°C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen im Färbe- und Waschechtheitsgerät Linitest Plus (Atlas) (60°C, 1h, 20rpm, Ariel Compact Pulverwaschmittel,10 Metallkugeln als Beiladung) und im Anschluss ein Spülvorgang (20°C, 15min, Leitungswasser).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät spectro-color (Dr. Lange).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

**Tabelle: Übersicht der Gelbwertreduktion -db [%] für den Einsatz von 1, 2 und 3 Additiven**

| **INCI** | **Beispiel [%]** | | | |
|---|---|---|---|---|
| | Bsp. 1 | Bsp. 2 | Bsp. 3 | Bsp. 4 |
| Piroctone | | 0,50 | | |
| Cellulose Gum | | | | 0,50 |
| Tetrasodium Iminodisuccinate | | | 0,50 | |
| Butylene Glycol Dicaprylate/Dicaprate | 3,00 | 3,00 | 3,00 | 3,00 |
| C18-36 Acid Triglyceride | 0,50 | 0,50 | 0,50 | 0,50 |
| Ceteareth-20 | 1,00 | 1,00 | 1,00 | 1,00 |
| VP/Hexadecene Copolymer | 1,00 | 1,00 | 1,00 | 1,00 |
| Perfume | q.s. | q.s. | q.s. | q.s. |
| Glycerin | 5,00 | 5,00 | 5,00 | 5,00 |
| Sodium Hydroxide | q.s. | q.s. | q.s. | q.s. |
| Phenoxyethanol | 0,50 | 0,50 | 0,50 | 0,50 |
| Ethylparaben | 0,20 | 0,20 | 0,20 | 0,20 |
| Methylparaben | 0,30 | 0,30 | 0,30 | 0,30 |
| Acrylates/C10-30 Alkyl Acrylate Crosspolymer | 0,50 | 0,50 | 0,50 | 0,50 |
| Alcohol Denat. | 4,00 | 4,00 | 4,00 | 4,00 |
| Trisodium EDTA | 0,20 | 0,20 | 0,20 | 0,20 |
| Homosalate | 9,00 | 9,00 | 9,00 | 9,00 |
| Octocrylene | 8,00 | 8,00 | 8,00 | 8,00 |
| Ethylhexyl Salicylate | 4,50 | 4,50 | 4,50 | 4,50 |
| Bis-Ethylhexyloxyphenol Methoxyphenyl Triazine | 4,00 | 4,00 | 4,00 | 4,00 |
| Butyl Methoxydibenzoylmethane | 4,50 | 4,50 | 4,50 | 4,50 |
| Phenylbenzimidazole Sulfonic Acid | 1,50 | 1,50 | 1,50 | 1,50 |
| Aqua | ad 100 | ad 100 | ad 100 | ad 100 |
| | | | | |

| Muster | Basis | 1 Additiv | 1 Additiv | 1 Additiv |
|---|---|---|---|---|
| Gelbreduktion -db [%] | - | -26 | -21% | -17% |

| **INCI** | **Beispiel [%]** | | | |
|---|---|---|---|---|
| | Bsp. 5 | Bsp. 6 | Bsp. 7 | Bsp. 8 |
| Piroctone | | 0,50 | 0,50 | |
| Cellulose Gum | 0,50 | 0,50 | 0,50 | 0,50 |
| Tetrasodium Iminodisuccinate | 0,50 | | | 0,50 |
| Sodium Diethylenetriamine Pentamethylene Phosphonate | | | 0,25 | 0,25 |
| Butylene Glycol Dicaprylate/Dicaprate | 3,00 | 3,00 | 3,00 | 3,00 |
| C18-36 Acid Triglyceride | 0,50 | 0,50 | 0,50 | 0,50 |
| Ceteareth-20 | 1,00 | 1,00 | 1,00 | 1,00 |
| VP/Hexadecene Copolymer | 1,00 | 1,00 | 1,00 | 1,00 |
| Perfume | q.s. | q.s. | q.s. | q.s. |
| Glycerin | 5,00 | 5,00 | 5,00 | 5,00 |
| Sodium Hydroxide | q.s. | q.s. | q.s. | q.s. |
| Phenoxyethanol | 0,50 | 0,50 | 0,50 | 0,50 |
| Ethylparaben | 0,20 | 0,20 | 0,20 | 0,20 |
| Methylparaben | 0,30 | 0,30 | 0,30 | 0,30 |
| Acrylates/C10-30 Alkyl Acrylate Crosspolymer | 0,50 | 0,50 | 0,50 | 0,50 |
| Alcohol Denat. | 4,00 | 4,00 | 4,00 | 4,00 |
| Trisodium EDTA | 0,20 | 0,20 | 0,20 | 0,20 |
| Homosalate | 9,00 | 9,00 | 9,00 | 9,00 |
| Octocrylene | 8,00 | 8,00 | 8,00 | 8,00 |
| Ethylhexyl Salicylate | 4,50 | 4,50 | 4,50 | 4,50 |
| Bis-Ethylhexyloxyphenol Methoxyphenyl Triazine | 4,00 | 4,00 | 4,00 | 4,00 |
| Butyl Methoxydibenzoylmethane | 4,50 | 4,50 | 4,50 | 4,50 |
| Phenylbenzimidazole Sulfonic Acid | 1,50 | 1,50 | 1,50 | 1,50 |
| Aqua | ad 100 | ad 100 | ad 100 | ad 100 |
| | | | | |
| Muster | 2 Additive | 2 Additive | 3 Additive | 3 Additive |
| Gelbreduktion -db [%] | -37 | -39 | -55 | -49 |

**Fazit:** Die erfindungsgemäße Stoffkombination führt zu einer deutlich stärkeren Reduzierung der Textilverfleckung als die Einzelstoffe oder die Zweistoff-Kombinationen.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **INCI** | **Beispiel [%]** | | | | | | | | | |
|---|---|---|---|---|---|---|---|---|---|---|
| | Bsp. 9 | Bsp. 10 | Bsp. 11 | Bsp. 12 | Bsp. 13 | Bsp. 14 | Bsp. 15 | Bsp. 16 | Bsp. 17 | Bsp. 18 |
| Triacontanyl PVP | 0,50 | | 1,00 | 1,00 | 1,00 | 1,00 | 1,00 | 1,00 | 1,00 | 1,00 |
| Cellulose Gum | 0,50 | 0,50 | 0,50 | 0,50 | 0,50 | 0,50 | 0,50 | 0,50 | 0,50 | 0,50 |
| Hydroxyacetophenone | 0,40 | 0,40 | | | | | 0,40 | | | |
| Panthenol | 1,00 | | 1,00 | | | | | | | |
| Ethylhexylglycerin | 0,30 | 0,30 | | 0,30 | | | 0,15 | | | |
| Piroctone Olamine | | | 0,10 | 0,10 | 0,45 | 0,10 | | 0,45 | | |
| C12-15 Alkyl Benzoate | 4,50 | 4,50 | 2,00 | 2,00 | | 2,00 | 2,00 | | | 2,00 |
| Myristyl Myristate | 0,50 | | | | 1,00 | | | | | |
| Hydrogenated Coco-Glycerides | 1,00 | 1,00 | | | | | | | | |
| Butylene Glycol Dicaprylate/Dicaprate | | 4,50 | 2,00 | 2,00 | | 2,00 | 2,00 | 2,00 | 2,00 | 2,00 |
| Dicaprylyl Carbonate | | 2,00 | | | | | | | | |
| C18-38 Alkyl Hydroxystearoyl Stearate | | | 1,00 | 1,00 | 0,50 | 1,00 | 1,00 | | | |
| Copernicia Cerifera Cera | | | | | | | | | 0,50 | |
| C18-36 Acid Triglyceride | | | | | | | | 1,00 | | 1,00 |
| Glyceryl Stearate Citrate | 2,00 | 2,00 | | | | | | | | |
| Glyceryl Stearate | | | 1,00 | 1,00 | | 1,00 | 1,00 | | | 1,00 |
| Sodium Stearoyl Glutamate | | | 0,30 | 0,30 | 0,00 | 0,30 | 0,25 | | | 0,40 |
| Glyceryl Stearate SE | | | | | 1,00 | | | | | |
| Sodium Cetearyl Sulfate | | | | | 0,15 | | | | | |
| Ceteareth-20 | | | | | | | | 1,50 | 1,50 | |
| Silica Dimethyl Silylate | | | 1,00 | 1,00 | 0,50 | 1,00 | 1,00 | | | |
| VP/Hexadecene Copolymer | | 0,50 | | | | | | | | |
| Parfum | q.s. | q.s. | q.s. | q.s. | q.s. | q.s. | q.s. | q.s. | q.s. | q.s. |
| Glycerin | 8,00 | 8,00 | 7,50 | 7,50 | 8,00 | 7,50 | 7,50 | 5,00 | 5,00 | 5,00 |
| Citric Acid | q.s. | q.s. | q.s. | q.s. | q.s. | q.s. | q.s. | q.s. | q.s. | q.s. |
| Sodium Hydroxide | q.s. | q.s. | q.s. | q.s. | q.s. | q.s. | q.s. | q.s. | q.s. | q.s. |
| Phenoxyethanol | | | 0,50 | 0,50 | 0,50 | 0,50 | | 0,50 | 0,50 | 0,50 |
| Methylparaben | | | | | 0,30 | | | 0,30 | 0,30 | 0,30 |
| Ethylparaben | | | | | | | | 0,20 | 0,20 | 0,20 |
| Stearyl Alcohol | 0,25 | | 1,00 | 1,00 | 1,00 | 1,00 | 1,25 | | | |
| Acrylates/C10-30 Alkyl Acrylate Crosspolymer | 0,15 | 0,15 | 0,15 | 0,15 | 0,10 | 0,15 | 0,15 | 0,35 | 0,45 | 0,10 |
| Xanthan Gum | 0,30 | 0,30 | 0,40 | 0,40 | 0,40 | 0,40 | 0,40 | | | 0,12 |
| Cetyl Alcohol | | 0,50 | | | | | | | | |
| Microcrystalline Cellulose | | | | | | | | | 1,00 | 1,00 |
| Aqua | q.s. | q.s. | q.s. | q.s. | q.s. | q.s. | q.s. | q.s. | q.s. | q.s. |
| Alcohol Denat. | 5,00 | 5,00 | 3,00 | 3,00 | 4,00 | 3,00 | 5,00 | 4,00 | 4,00 | 6,00 |
| Trisodium EDTA | 0,20 | 0,20 | 0,20 | 0,20 | 0,20 | 0,20 | 0,20 | 0,20 | 0,20 | 0,20 |
| Tetrasodium Iminodisuccinate | 0,75 | 0,75 | 0,75 | 0,75 | 0,00 | 0,75 | 0,75 | 0,00 | 0,75 | 0,75 |
| Sodium Diethylenetriamine Pentamethylene Phosphonate | | | 0,13 | 0,13 | 0,13 | 0,13 | 0,13 | 0,13 | 0,13 | 0,13 |
| Homosalate | 9,00 | 0,00 | 9,00 | 9,00 | 9,50 | 9,00 | 9,00 | 9,00 | 9,00 | 9,00 |
| Octocrylene | 9,00 | 9,00 | | | 9,50 | | | 8,00 | 8,00 | |
| Ethylhexyl Salicylate | 4,50 | 0,00 | 4,75 | 4,75 | 4,75 | 4,75 | 4,75 | 4,75 | 4,75 | 4,75 |
| Butyl Methoxydibenzoylmethane | 4,50 | 4,50 | 4,75 | 4,75 | 4,75 | 4,75 | 4,75 | 4,50 | 4,50 | 4,75 |
| Titanium Dioxide | 3,00 | 6,00 | | | | | 1,00 | | | |
| Bis-Ethylhexyloxyphenol Methoxyphenyl Triazine | | 3,00 | 4,00 | 4,00 | 3,50 | 4,00 | 4,00 | 3,50 | 3,00 | 4,00 |
| Ethylhexyl Triazone | | | 3,00 | 3,00 | | 3,00 | 3,00 | | | 3,00 |
| Phenylbenzimidazole Sulfonic Acid | | | 1,00 | 1,00 | 1,00 | 1,00 | 1,00 | 1,50 | 1,50 | 1,00 |

## Patentansprüche

1. Kosmetische Zubereitung enthaltend
4-(tert.-Butyl)-4'-methoxydibenzoylmethan, Cellulose Gum und
mindestens ein Additiv gewählt aus der Gruppe der Verbindungen Iminodisuccinat, 1-Hydroxy-4-methyl-6-(2,4,4-trimethylpentyl)-2(1H)-pyridon (Pirocton) und Diethylentriaminpenta(methylenphosphonsäure)/ DTPMP, wobei die Rezepturen
| | Gew.-% | Gew.-% | Gew.-% |
|---|---|---|---|
| Carboxymethylcellulose | | 0,75 | 0,5 |
| Cellulose Gum | 1,0 | 0,75 | 0,75 |
| Trisodium EDTA | 0,20 | 0,20 | 0,50 |
| Tetranatrium im inodisuccinate | 0,75 | 0,50 | |
| Diethylentriaminpenta(methylenphosphonsäure) | | 0,50 | 0,50 |
| Butylmethoxydibenzoylmethane | 5,00 | 4,00 | 3,00 |
| Bis-Ethylhexyloxyphenol Methoxyphenyl Triazine | 3,50 | 3,50 | 0,50 |
| Diethylamino Hydroxybenzoyl Hexylbenzoate | 0,50 | 1,00 | |
| Phenylbenzimidazole Sulfonic Acid | | 1,00 | 1,00 |
| Ethylhexyl Salicylate | 4,50 | 5,00 | 5,00 |
| Titanium Dioxide | 3,00 | 2,00 | |
| Trimethoxycaprylylsilane | 0,20 | 0,20 | |
| Octocrylene | 10,00 | 10,00 | 10,00 |
| Homosalate | 4,50 | 10,00 | 10,00 |
| Cetearyl Alcohol | | 1,00 | 0,50 |
| Xantham. Gum | 0,40 | 0,40 | 0,40 |
| Acrylates/C10-30 Alkyl Acrylate Crosspolymer | 0,30 | 0,05 | 0,20 |
| Alcohol denat. | 5,00 | 4,00 | 6,00 |
| Methylparaben | 0,30 | 0,30 | 0,30 |
| Phenoxyethanol | 0,60 | 0,60 | 0,60 |
| Citric Acid | 0,30 | | |
| Sodium Citrate | 0,10 | | |
| Sodium Hydroxide | 0,20 | 0,40 | 0,50 |
| Glycerin | 3,00 | 9,00 | 3,00 |
| Parfum | 0,50 | 0,40 | 0,60 |
| VP/Hexadecene Copolymer | 0,50 | 0,50 | |
| Silica Dimethyl Silylate | | 0,50 | 0,50 |
| Sodium Cetearyl Sulfate | | 0,15 | |
| Glyceryl Stearate SE | | 1,00 | |
| Glyceryl Stearate Citrate | 2,00 | | |
| Sodium Stearoyl Glutamate | | | 0,40 |
| Glyceryl Stearate | | | 1,00 |
| Hydrogenated Coco-Glycerides | 1,00 | | 1,00 |
| C12-15 Alkyl Benzoate | 5,00 | | |
| Myristyl Myristate | 1,00 | 1,00 | |
| Stearyl Alcohol | 0,50 | | |
| C18-38 Alkyl Hydroxystearoyl Stearate | | 0,50 | |
| Isopropyl Stearate | 2,00 | | |
| Butylene Glycol Dicaprylate/Dicaprate | 5,00 | | |
| Aqua | Ad. 100 | Ad. 100 | Ad. 100 |
vom Schutzbereich ausgenommen sind.

2. Kosmetische Zubereitung nach Anspruch 1, **dadurch gekennzeichnet, dass** die Zubereitung4-(tert.-Butyl)-4'-methoxydibenzoylmethan in einer Menge von 0,01 bis 5 Gewichts-%, bezogen auf das Gesamtgewicht der Zubereitung, enthält.

3. Kosmetische Zubereitung nach einem der vorhergehenden Ansprüche, **dadurch gekennzeichnet, dass** die Zubereitung Cellulose Gum in einer Konzentration von 0,01 bis 2 Gewichts-%, bezogen auf das Gesamtgewicht der Zubereitung, enthält.

4. Kosmetische Zubereitung nach einem der vorhergehenden Ansprüche, **dadurch gekennzeichnet, dass** die Zubereitung Iminodisuccinat in einer Konzentration von 0,01 bis 2 Gewichts-%, bezogen auf das Gesamtgewicht der Zubereitung, enthält.

5. Kosmetische Zubereitung nach einem der vorhergehenden Ansprüche, **dadurch gekennzeichnet, dass** die Zubereitung Diethylentriaminpenta(methylenphosphonsäure)/ DTPMP in einer Konzentration von 0,01 bis 1 Gewichts-%, bezogen auf das Gesamtgewicht der Zubereitung, enthält.

6. Kosmetische Zubereitung nach einem der vorhergehenden Ansprüche, **dadurch gekennzeichnet, dass** die Zubereitung 1-Hydroxy-4-methyl-6-(2,4,4-trimethylpentyl)-2(1H)-pyridon (Pirocton) in einer Gesamtmenge von 0,01 bis 1 Gewichts-%, bezogen auf das Gesamtgewicht der Zubereitung, enthält.

7. Kosmetische Zubereitung nach einem der vorhergehenden Ansprüche, **dadurch gekennzeichnet, dass** das1-Hydroxy-4-methyl-6-(2,4,4-trimethylpentyl)-2(1H)-pyridon (Pirocton) in Form seines Monoethanolamin-Salzes (Piroctone Olamine) eingesetzt wird.

8. Kosmetische Zubereitung nach einem der vorhergehenden Ansprüche, **dadurch gekennzeichnet, dass** die Zubereitung einen oder mehrere Emulgatoren gewählt aus der Gruppe der Verbindungen Glycerylstearatcitrat, Cetearylalkohol, Natriumcetearylsulfat, Glycerylstearat, Cetearylsulfosuccinat, Natriumstearoylglutamat, Polyglyceryl-3-methylglucosedistearat, Stearinsäure, Polyglyceryl-10 Stearat, Kaliumcetylphosphat, enthält.

9. Kosmetische Zubereitung nach einem der vorhergehenden Ansprüche, **dadurch gekennzeichnet, dass** die Zubereitung einen oder mehrere UV-Filterenthält , die gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäureamylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzälmalonat; 3-(4-(2,2-bis Ethoxycarbonylvinyl)-phenoxy)propenyl)-methoxysiloxan / Dimethylsiloxan - Copolymer; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethylhexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

10. Kosmetische Zubereitung nach einem der vorhergehenden Ansprüche, **dadurch gekennzeichnet, dass** die Zubereitung eine oder mehrere Verbindungen gewählt aus der Gruppe der Verbindungen Ethylhexylglycerin, Polyglyceryl-2 Caprate, Propylenglycol, Butylenglycol, 2-Methylpropan-1,3-diol, 1,2-Pentandiol, 1,2-Hexandiol, 1,2-Octandiol und/oder 1,2-Decandiol enthält.

11. Kosmetische Zubereitung nach einem der vorhergehenden Ansprüche, **dadurch gekennzeichnet, dass** die Zubereitung Phenoxyethanol und/oder Methylparaben enthält.

12. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen Magnolienextrakt, alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, β-Alanin, Tocopherylacetat, Panthenol, Magnolol, Honokiol, Harnstoff, Hyaluronsäure, Dihydroxyaceton, 8-Hexadecen-1,16-dicarbonsäure, Glycyrrhetinsäure, Glucosylglyceride und/oder Licochalcon A enthält.

13. Kosmetische Zubereitung nach einem der vorhergehenden Ansprüche, **dadurch gekennzeichnet, dass** die Zubereitung frei ist von Propyl- und Butylparaben, 3-lod-2-propinylbutylcarbamat, 3-(4-Methylbenzyliden)-campher und 2-Hydroxy-4-methoxybenzophenon (Oxybenzon).

14. Kosmetische Zubereitung nach einem der vorhergehenden Ansprüche, **dadurch gekennzeichnet, dass** die Zubereitung C12-15 Alkylbenzoat, Cyclomethicon, Octyldodecanol, Capryl/Caprinsäure Triglyceride, Butylenglykol Dicaprylat/Dicaprat, Phenethylbenzoat, Cocoglyceride, Di-n-butyladipat und/oder Diisopropyladipat enthält.

15. Verfahren zur Erleichterung der Auswaschbarkeit von 4-(tert.-Butyl)-4'-methoxydibenzoylmethan enthaltenden kosmetischen Zubereitungen aus Textilien, **dadurch gekennzeichnet, dass** dem Kosmetikum Cellulose Gum und mindestens ein Additiv gewählt aus der Gruppe der Verbindungen Iminodisuccinat, 1-Hydroxy-4-methyl-6-(2,4,4-trimethylpentyl)-2(1H)-pyridon (Pirocton) und Diethylentriaminpenta(methylenphosphonsäure)/ DTPMP zugesetzt werden wobei Verfahren mit den Rezepturen
| | Gew.-% | Gew.-% | Gew.-% |
|---|---|---|---|
| Carboxymethylcellulose | | 0,75 | 0,5 |
| Cellulose Gum | 1,0 | 0,75 | 0,75 |
| Trisodium EDTA | 0,20 | 0,20 | 0,50 |
| Tetranatriumiminodisuccinate | 0,75 | 0,50 | |
| Diethylentriaminpenta(methylenphosphonsäure) | | 0,50 | 0,50 |
| Butylmethoxydibenzoylmethane | 5,00 | 4,00 | 3,00 |
| Bis-Ethylhexyloxyphenol Methoxyphenyl Triazine | 3,50 | 3,50 | 0,50 |
| Diethylamino Hydroxybenzoyl Hexylbenzoate | 0,50 | 1,00 | |
| Phenylbenzimidazole Sulfonic Acid | | 1,00 | 1,00 |
| Ethylhexyl Salicylate | 4,50 | 5,00 | 5,00 |
| Titanium Dioxide | 3,00 | 2,00 | |
| Trimethoxycaprylylsilane | 0,20 | 0,20 | |
| Octocrylene | 10,00 | 10,00 | 10,00 |
| Homosalate | 4,50 | 10,00 | 10,00 |
| Cetearyl Alcohol | | 1,00 | 0,50 |
| Xantham Gum | 0,40 | 0,40 | 0,40 |
| Acrylates/C10-30 Alkyl Acrylate Crosspolymer | 0,30 | 0,05 | 0,20 |
| Alcohol denat. | 5,00 | 4,00 | 6,00 |
| Methylparaben | 0,30 | 0,30 | 0,30 |
| Phenoxyethanol | 0,60 | 0,60 | 0,60 |
| Citric Acid | 0,30 | | |
| Sodium Citrate | 0,10 | | |
| Sodium Hydroxide | 0,20 | 0,40 | 0,50 |
| Glycerin | 3,00 | 9,00 | 3,00 |
| Parfum | 0,50 | 0,40 | 0,60 |
| VP/Hexadecene Copolymer | 0,50 | 0,50 | |
| Silica Dimethyl Silylate | | 0,50 | 0,50 |
| Sodium Cetearyl Sulfate | | 0,15 | |
| Glyceryl Stearate SE | | 1,00 | |
| Glyceryl Stearate Citrate | 2,00 | | |
| Sodium Stearoyl Glutamate | | | 0,40 |
| Glyceryl Stearate | | | 1,00 |
| Hydrogenated Coco-Glycerides | 1,00 | | 1,00 |
| C12-15 Alkyl Benzoate | 5,00 | | |
| Myristyl Myristate | 1,00 | 1,00 | |
| Stearyl Alcohol | 0,50 | | |
| C18-38 Alkyl Hydroxystearoyl Stearate | | 0,50 | |
| Isopropyl Stearate | 2,00 | | |
| Butylene Glycol Dicaprylate/Dicaprate | 5,00 | | |
| Aqua | Ad. 100 | Ad. 100 | Ad. 100 |
vom Schutzbereich ausgenommen sind.

16. Verwendung von Cellulose Gum und mindestens einem Additiv gewählt aus der Gruppe der Verbindungen Iminodisuccinat, 1-Hydroxy-4-methyl-6-(2,4,4-trimethylpentyl)-2(1H)-pyridon (Pirocton) und Diethylentriaminpenta(methylenphosphonsäure)/ DTPMP in 4-(tert.-Butyl)-4'-methoxydibenzoylmethan enthaltenden kosmetischen Zubereitungen zur Erleichterung der Auswaschbarkeit der UV-Lichtschutzfilter aus mit den Zubereitungen kontaminierten Textilien, wobei die Rezepturen
| | Gew.-% | Gew.-% | Gew.-% |
|---|---|---|---|
| Carboxymethylcellulose | | 0,75 | 0,5 |
| Cellulose Gum | 1,0 | 0,75 | 0,75 |
| Trisodium EDTA | 0,20 | 0,20 | 0,50 |
| Tetranatrium im inodisuccinate | 0,75 | 0,50 | |
| Diethylentriaminpenta(methylenphosphonsäure) | | 0,50 | 0,50 |
| Butylmethoxydibenzoylmethane | 5,00 | 4,00 | 3,00 |
| Bis-Ethylhexyloxyphenol Methoxyphenyl Triazine | 3,50 | 3,50 | 0,50 |
| Diethylamino Hydroxybenzoyl Hexylbenzoate | 0,50 | 1,00 | |
| Phenylbenzimidazole Sulfonic Acid | | 1,00 | 1,00 |
| Ethylhexyl Salicylate | 4,50 | 5,00 | 5,00 |
| Titanium Dioxide | 3,00 | 2,00 | |
| Trimethoxycaprylylsilane | 0,20 | 0,20 | |
| Octocrylene | 10,00 | 10,00 | 10,00 |
| Homosalate | 4,50 | 10,00 | 10,00 |
| Cetearyl Alcohol | | 1,00 | 0,50 |
| Xantham Gum | 0,40 | 0,40 | 0,40 |
| Acrylates/C10-30 Alkyl Acrylate Crosspolymer | 0,30 | 0,05 | 0,20 |
| Alcohol denat. | 5,00 | 4,00 | 6,00 |
| Methylparaben | 0,30 | 0,30 | 0,30 |
| Phenoxyethanol | 0,60 | 0,60 | 0,60 |
| Citric Acid | 0,30 | | |
| Sodium Citrate | 0,10 | | |
| Sodium Hydroxide | 0,20 | 0,40 | 0,50 |
| Glycerin | 3,00 | 9,00 | 3,00 |
| Parfum | 0,50 | 0,40 | 0,60 |
| VP/Hexadecene Copolymer | 0,50 | 0,50 | |
| Silica Dimethyl Silylate | | 0,50 | 0,50 |
| Sodium Cetearyl Sulfate | | 0,15 | |
| Glyceryl Stearate SE | | 1,00 | |
| Glyceryl Stearate Citrate | 2,00 | | |
| Sodium Stearoyl Glutamate | | | 0,40 |
| Glyceryl Stearate | | | 1,00 |
| Hydrogenated Coco-Glycerides | 1,00 | | 1,00 |
| C12-15 Alkyl Benzoate | 5,00 | | |
| Myristyl Myristate | 1,00 | 1,00 | |
| Stearyl Alcohol | 0,50 | | |
| C18-38 Alkyl Hydroxystearoyl Stearate | | 0,50 | |
| Isopropyl Stearate | 2,00 | | |
| Butylene Glycol Dicaprylate/Dicaprate | 5,00 | | |
| Aqua | Ad. 100 | Ad. 100 | Ad. 100 |
vom Schutzbereich ausgenommen sind.

## Claims

1. Cosmetic preparation comprising 4-(tert-butyl)-4'-methoxydibenzoylmethane, cellulose gum and at least one additive selected from the group of compounds comprising iminodisuccinate, 1-hydroxy-4-methyl-6-(2,4,4-trimethylpentyl)-2(1H)-pyridone (piroctone) and diethylenetriamine penta(methylene phosphonic acid)/DTPMP, wherein the formulations
| | wt% | wt% | wt% |
|---|---|---|---|
| Carboxymethylcellulose | | 0.75 | 0.5 |
| Cellulose Gum | 1.0 | 0.75 | 0.75 |
| Trisodium EDTA | 0.20 | 0.20 | 0.50 |
| Tetrasodium iminodisuccinate | 0.75 | 0.50 | |
| Diethylenetriamine penta(methylene phosphonic acid) | | 0.50 | 0.50 |
| Butylmethoxydibenzoylmethane | 5.00 | 4.00 | 3.00 |
| Bis-Ethylhexyloxyphenol Methoxyphenyl Triazine | 3.50 | 3.50 | 0.50 |
| Diethylamino Hydroxybenzoyl Hexylbenzoate | 0.50 | 1.00 | |
| Phenylbenzimidazole Sulfonic Acid | | 1.00 | 1.00 |
| Ethylhexyl Salicylate | 4.50 | 5.00 | 5.00 |
| Titanium Dioxide | 3.00 | 2.00 | |
| Trimethoxycaprylylsilane | 0.20 | 0.20 | |
| Octocrylene | 10.00 | 10.00 | 10.00 |
| Homosalate | 4.50 | 10.00 | 10.00 |
| Cetearyl Alcohol | | 1.00 | 0.50 |
| Xanthan Gum | 0.40 | 0.40 | 0.40 |
| Acrylates/C10-30 Alkyl Acrylate Crosspolymer | 0.30 | 0.05 | 0.20 |
| Alcohol denat. | 5.00 | 4.00 | 6.00 |
| Methylparaben | 0.30 | 0.30 | 0.30 |
| Phenoxyethanol | 0.60 | 0.60 | 0.60 |
| Citric Acid | 0.30 | | |
| Sodium Citrate | 0.10 | | |
| Sodium Hydroxide | 0.20 | 0.40 | 0.50 |
| Glycerin | 3.00 | 9.00 | 3.00 |
| Perfume | 0.50 | 0.40 | 0.60 |
| VP/Hexadecene Copolymer | 0.50 | 0.50 | |
| Silica Dimethyl Silylate | | 0.50 | 0.50 |
| Sodium Cetearyl Sulfate | | 0.15 | |
| Glyceryl Stearate SE | | 1.00 | |
| Glyceryl Stearate Citrate | 2.00 | | |
| Sodium Stearoyl Glutamate | | | 0.40 |
| Glyceryl Stearate | | | 1.00 |
| Hydrogenated Coco-Glycerides | 1.00 | | 1.00 |
| C12-15 Alkyl Benzoate | 5.00 | | |
| Myristyl Myristate | 1.00 | 1.00 | |
| Stearyl Alcohol | 0.50 | | |
| C18-38 Alkyl Hydroxystearoyl Stearate | | 0.50 | |
| Isopropyl Stearate | 2.00 | | |
| Butylene Glycol Dicaprylate/Dicaprate | 5.00 | | |
| Aqua | to 100 | to 100 | to 100 |
are excluded from the scope of protection.

2. Cosmetic preparation according to Claim 1, **characterized in that** the preparation comprises 4-(tert-butyl)-4'-methoxydibenzoylmethane in an amount of 0.01 to 5% by weight, based on the total weight of the preparation.

3. Cosmetic preparation according to either of the preceding claims, **characterized in that** the preparation comprises cellulose gum at a concentration of 0.01 to 2% by weight, based on the total weight of the preparation.

4. Cosmetic preparation according to any of the preceding claims, **characterized in that** the preparation comprises iminodisuccinate at a concentration of 0.01 to 2% by weight, based on the total weight of the preparation.

5. Cosmetic preparation according to any of the preceding claims, **characterized in that** the preparation comprises diethylenetriamine penta(methylene phosphonic acid)/DTPMP at a concentration of 0.01 to 1% by weight, based on the total weight of the preparation.

6. Cosmetic preparation according to any of the preceding claims, **characterized in that** the preparation comprises 1-hydroxy-4-methyl-6-(2,4,4-trimethylpentyl)-2(1H)-pyridone (piroctone) in a total amount of 0.01 to 1% by weight, based on the total weight of the preparation.

7. Cosmetic preparation according to any of the preceding claims, **characterized in that** the 1-hydroxy-4-methyl-6-(2,4,4-trimethylpentyl)-2(1H)-pyridone (piroctone) is used in the form of its monoethanolamine salt (piroctone olamine).

8. Cosmetic preparation according to any of the preceding claims, **characterized in that** the preparation comprises one or more emulsifiers selected from the group of compounds comprising glyceryl stearate citrate, cetearyl alcohol, sodium cetearyl sulfate, glyceryl stearate, cetearyl sulfosuccinate, sodium stearoyl glutamate, polyglyceryl-3 methylglucose distearate, stearic acid, polyglyceryl-10 stearate, potassium cetyl phosphate.

9. Cosmetic preparation according to any of the preceding claims, **characterized in that** the preparation comprises one or more UV filters selected from the group of compounds comprising 2-phenylbenzimidazole-5-sulfonic acid and/or salts thereof; phenylene-1,4-bis(2-benzimidazyl)-3,3'-5,5'-tetrasulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulfonic acid; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxysiloxane/dimethylsiloxane - copolymer; hexyl 2-(4'-diethylamino-2'-hydroxybenzoyl)benzoate; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4,6-tribiphenyl-4-yl-1,3,5-triazine; merocyanine; titanium dioxide; zinc oxide.

10. Cosmetic preparation according to any of the preceding claims, **characterized in that** the preparation comprises one or more compounds selected from the group of compounds comprising ethylhexylglycerin, polyglyceryl-2 caprate, propylene glycol, butylene glycol, 2-methylpropane-1,3-diol, pentane-1,2-diol, hexane-1,2-diol, octane-1,2-diol and/or decane-1,2-diol.

11. Cosmetic preparation according to any of the preceding claims, **characterized in that** the preparation comprises phenoxyethanol and/or methylparaben.

12. Cosmetic preparation according to any of the preceding claims, **characterized in that** the preparation comprises one or more active ingredients selected from the group of compounds comprising magnolia extract, alpha-lipoic acid, folic acid, phytoene, D-biotin, coenzyme Q10, alpha-glucosylrutin, carnitine, carnosine, polydocanol, natural and/or synthetic isoflavonoids, flavonoids, creatine, creatinine, taurine, β-alanine, tocopheryl acetate, panthenol, magnolol, honokiol, urea, hyaluronic acid, dihydroxyacetone, 8-hexadecene-1,16-dicarboxylic acid, glycyrrhetic acid, glucosyl glycerides and/or licochalcone A.

13. Cosmetic preparation according to any of the preceding claims, **characterized in that** the preparation is free from propylparaben and butylparaben, 3-iodo-2-propynyl butylcarbamate, 3-(4-methylbenzylidene)camphor and 2-hydroxy-4-methoxybenzophenone (Oxybenzone).

14. Cosmetic preparation according to any of the preceding claims, **characterized in that** the preparation comprises C12-15 alkyl benzoate, cyclomethicone, octyldodecanol, caprylic/capric triglycerides, butylene glycol dicaprylate/dicaprate, phenethyl benzoate, coco-glycerides, di-n-butyl adipate and/or diisopropyl adipate.

15. Process for facilitating the ability of cosmetic preparations comprising 4-(tert-butyl)-4'-methoxydibenzoylmethane to be washed out of textiles, **characterized in that** cellulose gum and at least one additive selected from the group of compounds comprising iminodisuccinate, 1-hydroxy-4-methyl-6-(2,4,4-trimethylpentyl)-2(1H)-pyridone (piroctone) and diethylenetriamine penta(methylene phosphonic acid)/DTPMP are added to the cosmetic wherein processes with the formulations
| | wt% | wt% | wt% |
|---|---|---|---|
| Carboxymethylcellulose | | 0.75 | 0.5 |
| Cellulose Gum | 1.0 | 0.75 | 0.75 |
| Trisodium EDTA | 0.20 | 0.20 | 0.50 |
| Tetrasodium iminodisuccinate | 0.75 | 0.50 | |
| Diethylenetriamine penta(methylene phosphonic acid) | | 0.50 | 0.50 |
| Butylmethoxydibenzoylmethane | 5.00 | 4.00 | 3.00 |
| Bis-Ethylhexyloxyphenol Methoxyphenyl Triazine | 3.50 | 3.50 | 0.50 |
| Diethylamino Hydroxybenzoyl Hexylbenzoate | 0.50 | 1.00 | |
| Phenylbenzimidazole Sulfonic Acid | | 1.00 | 1.00 |
| Ethylhexyl Salicylate | 4.50 | 5.00 | 5.00 |
| Titanium Dioxide | 3.00 | 2.00 | |
| Trimethoxycaprylylsilane | 0.20 | 0.20 | |
| Octocrylene | 10.00 | 10.00 | 10.00 |
| Homosalate | 4.50 | 10.00 | 10.00 |
| Cetearyl Alcohol | | 1.00 | 0.50 |
| Xanthan Gum | 0.40 | 0.40 | 0.40 |
| Acrylates/C10-30 Alkyl Acrylate Crosspolymer | 0.30 | 0.05 | 0.20 |
| Alcohol denat. | 5.00 | 4.00 | 6.00 |
| Methylparaben | 0.30 | 0.30 | 0.30 |
| Phenoxyethanol | 0.60 | 0.60 | 0.60 |
| Citric Acid | 0.30 | | |
| Sodium Citrate | 0.10 | | |
| Sodium Hydroxide | 0.20 | 0.40 | 0.50 |
| Glycerin | 3.00 | 9.00 | 3.00 |
| Perfume | 0.50 | 0.40 | 0.60 |
| VP/Hexadecene Copolymer | 0.50 | 0.50 | |
| Silica Dimethyl Silylate | | 0.50 | 0.50 |
| Sodium Cetearyl Sulfate | | 0.15 | |
| Glyceryl Stearate SE | | 1.00 | |
| Glyceryl Stearate Citrate | 2.00 | | |
| Sodium Stearoyl Glutamate | | | 0.40 |
| Glyceryl Stearate | | | 1.00 |
| Hydrogenated Coco-Glycerides | 1.00 | | 1.00 |
| C12-15 Alkyl Benzoate | 5.00 | | |
| Myristyl Myristate | 1.00 | 1.00 | |
| Stearyl Alcohol | 0.50 | | |
| C18-38 Alkyl Hydroxystearoyl Stearate | | 0.50 | |
| Isopropyl Stearate | 2.00 | | |
| Butylene Glycol Dicaprylate/Dicaprate | 5.00 | | |
| Aqua | to 100 | to 100 | to 100 |
are excluded from the scope of protection.

16. Use of cellulose gum and at least one additive selected from the group of compounds comprising iminodisuccinate, 1-hydroxy-4-methyl-6-(2,4,4-trimethylpentyl)-2(1H)-pyridone (piroctone) and diethylenetriamine penta(methylene phosphonic acid)/DTPMP in cosmetic formulations comprising 4-(tert-butyl)-4'-methoxydibenzoylmethane for facilitating the ability of UV light protection filters to be washed out of textiles contaminated with the preparations, wherein the formulations
| | wt% | wt% | wt% |
|---|---|---|---|
| Carboxymethylcellulose | | 0.75 | 0.5 |
| Cellulose Gum | 1.0 | 0.75 | 0.75 |
| Trisodium EDTA | 0.20 | 0.20 | 0.50 |
| Tetrasodium iminodisuccinate | 0.75 | 0.50 | |
| Diethylenetriamine penta(methylene phosphonic acid) | | 0.50 | 0.50 |
| Butylmethoxydibenzoylmethane | 5.00 | 4.00 | 3.00 |
| Bis-Ethylhexyloxyphenol Methoxyphenyl Triazine | 3.50 | 3.50 | 0.50 |
| Diethylamino Hydroxybenzoyl Hexylbenzoate | 0.50 | 1.00 | |
| Phenylbenzimidazole Sulfonic Acid | | 1.00 | 1.00 |
| Ethylhexyl Salicylate | 4.50 | 5.00 | 5.00 |
| Titanium Dioxide | 3.00 | 2.00 | |
| Trimethoxycaprylylsilane | 0.20 | 0.20 | |
| Octocrylene | 10.00 | 10.00 | 10.00 |
| Homosalate | 4.50 | 10.00 | 10.00 |
| Cetearyl Alcohol | | 1.00 | 0.50 |
| Xanthan Gum | 0.40 | 0.40 | 0.40 |
| Acrylates/C10-30 Alkyl Acrylate Crosspolymer | 0.30 | 0.05 | 0.20 |
| Alcohol denat. | 5.00 | 4.00 | 6.00 |
| Methylparaben | 0.30 | 0.30 | 0.30 |
| Phenoxyethanol | 0.60 | 0.60 | 0.60 |
| Citric Acid | 0.30 | | |
| Sodium Citrate | 0.10 | | |
| Sodium Hydroxide | 0.20 | 0.40 | 0.50 |
| Glycerin | 3.00 | 9.00 | 3.00 |
| Perfume | 0.50 | 0.40 | 0.60 |
| VP/Hexadecene Copolymer | 0.50 | 0.50 | |
| Silica Dimethyl Silylate | | 0.50 | 0.50 |
| Sodium Cetearyl Sulfate | | 0.15 | |
| Glyceryl Stearate SE | | 1.00 | |
| Glyceryl Stearate Citrate | 2.00 | | |
| Sodium Stearoyl Glutamate | | | 0.40 |
| Glyceryl Stearate | | | 1.00 |
| Hydrogenated Coco-Glycerides | 1.00 | | 1.00 |
| C12-15 Alkyl Benzoate | 5.00 | | |
| Myristyl Myristate | 1.00 | 1.00 | |
| Stearyl Alcohol | 0.50 | | |
| C18-38 Alkyl Hydroxystearoyl Stearate | | 0.50 | |
| Isopropyl Stearate | 2.00 | | |
| Butylene Glycol Dicaprylate/Dicaprate | 5.00 | | |
| Aqua | to 100 | to 100 | to 100 |
are excluded from the scope of protection.

## Revendications

1. Préparation cosmétique contenant du 4-(tert-butyl)-4'-méthoxydibenzoylméthane, une gomme de cellulose et au moins un additif choisi dans le groupe de composés formé par l'iminodisuccinate, la 1-hydroxy-4-méthyl-6-(2,4,4-triméthylpentyl)-2(1H)-pyridone (piroctone) et l'acide diéthylènetriaminepenta(méthylènephosphonique)/ DTPMP les formulations
| | % en poids | % en poids | % en poids |
|---|---|---|---|
| Carboxyméthylcellulose | | 0,75 | 0,5 |
| Gomme de cellulose | 1,0 | 0,75 | 0,75 |
| EDTA trisodique | 0,20 | 0,20 | 0,50 |
| Iminodisuccinate tétrasodique | 0,75 | 0,50 | |
| acide diéthylènetriaminepenta(méthylènephosphonique) | | 0,50 | 0,50 |
| Butylméthoxydibenzoylméthane | 5,00 | 4,00 | 3,00 |
| Bis-éthylhexyloxyphénol-méthoxyphényltriazine | 3,50 | 3,50 | 0,50 |
| Diéthylaminohydroxybenzoylbenzoate d'hexyle | 0,50 | 1,00 | |
| Acide phénylbenzimidazolsulfonique | | 1,00 | 1,00 |
| Salicylate d'éthylhexyle | 4,50 | 5,00 | 5,00 |
| Dioxyde de titane | 3,00 | 2,00 | |
| Triméthoxycaprylylsilane | 0,20 | 0,20 | |
| Octocrylène | 10,00 | 10,00 | 10,00 |
| Homosalate | 4,50 | 10,00 | 10,00 |
| Alcool cétéarylique | | 1,00 | 0,50 |
| Gomme xanthane | 0,40 | 0,40 | 0,40 |
| Polymère croisé acrylates/acrylate d'alkyle en C10 à C30 | 0,30 | 0,05 | 0,20 |
| Alcool dénaturé | 5,00 | 4,00 | 6,00 |
| Méthylparabène | 0,30 | 0,30 | 0,30 |
| Phénoxyéthanol | 0,60 | 0,60 | 0,60 |
| Acide citrique | 0,30 | | |
| Citrate de sodium | 0,10 | | |
| Hydroxyde de sodium | 0,20 | 0,40 | 0,50 |
| Glycérine | 3,00 | 9,00 | 3,00 |
| Parfum | 0,50 | 0,40 | 0,60 |
| Copolymère VP/hexadécène | 0,50 | 0,50 | |
| Diméthylsilylate de silice | | 0,50 | 0,50 |
| Cétéarylsulfate de sodium | | 0,15 | |
| Stéarate de glycéryle SE | | 1,00 | |
| Stéarate citrate de glycéryle | 2,00 | | |
| Stéaroylglutamate de sodium | | | 0,40 |
| Stéarate de glycéryle | | | 1,00 |
| Coco-glycérides hydrogénés | 1,00 | | 1,00 |
| Benzoate d'alkyle en C12 à C15 | 5,00 | | |
| Myristate de myristyle | 1,00 | 1,00 | |
| Alcool stéarylique | 0,50 | | |
| Hydroxystéaroylstéarate d'alkyle en C18 à C38 | | 0,50 | |
| Stéarate d'isopropyle | 2,00 | | |
| Dicaprylate/dicaprate de butylèneglycol | 5,00 | | |
| Eau | qsp 100 | qsp 100 | qsp 100 |
étant exclues du domaine de protection.

2. Préparation cosmétique selon la revendication 1, **caractérisée en ce que** la préparation contient du 4-(tert-butyl)-4'-méthoxydibenzoylméthane en une quantité de 0,01 à 5 % en poids, par rapport au poids total de la préparation.

3. Préparation cosmétique selon l'une quelconque des revendications précédentes, **caractérisée en ce que** la préparation contient une gomme de cellulose en une concentration de 0,01 à 2 % en poids, par rapport au poids total de la préparation.

4. Préparation cosmétique selon l'une quelconque des revendications précédentes, **caractérisée en ce que** la préparation contient de l'iminodisuccinate en une concentration de 0,01 à 2 % en poids, par rapport au poids total de la préparation.

5. Préparation cosmétique selon l'une quelconque des revendications précédentes, **caractérisée en ce que** la préparation contient de l'acide diéthylènetriaminepenta(méthylènephosphonique)/ DTPMP en une concentration de 0,01 à 1 % en poids, par rapport au poids total de la préparation.

6. Préparation cosmétique selon l'une quelconque des revendications précédentes, **caractérisée en ce que** la préparation contient de la 1-hydroxy-4-méthyl-6-(2,4,4-triméthylpentyl)-2(1H)-pyridone (piroctone) en une quantité totale de 0,01 à 1 % en poids, par rapport au poids total de la préparation.

7. Préparation cosmétique selon l'une quelconque des revendications précédentes, **caractérisée en ce que** la 1-hydroxy-4-méthyl-6-(2,4,4-triméthylpentyl)-2(1H)-pyridone (piroctone) est utilisée sous la forme de son sel avec la monoéthanolamine (Piroctone Olamine).

8. Préparation cosmétique selon l'une quelconque des revendications précédentes, **caractérisée en ce que** la préparation contient un ou plusieurs émulsifiants choisis dans le groupe de composés formé par le stéarate citrate de glycéryle, l'alcool cétéarylique, le cétéarylsulfate de sodium, le stéarate de glycéryle, le sulfosuccinate de cétéaryle, le stéaroylglutamate de sodium, le 3-méthylglucosedistéarate de polyglycéryle, l'acide stéarique, le stéarate de polyglycéryl-10, le cétylphosphate de potassium.

9. Préparation cosmétique selon l'une quelconque des revendications précédentes, **caractérisée en ce que** la préparation contient un ou plusieurs filtres UV, qui sont choisis dans le groupe de composés formé par l'acide 2-phénylbenzimidazole-5-sulfonique et/ou ses sels ; les sels d'acide phénylène-1,4-bis-(2-benzimidazyl)-3,3'-5,5'-tétrasulfonique ; le 1,4-di(2-oxo-10-sulfo-3-bornylidèneméthyl)-benzène et ses sels ; les sels d'acide 4-(2-oxo-3-bornylidèneméthyl)benzènesulfonique ; les sels de l'acide 2-méthyl-5-(2-oxo-3-bornylidèneméthyl)sulfonique ; le 2,2'-méthylène-bis-(6-(2H-benzotriazol-2-yl)-4-(1,1,3,3-tétraméthylbutyl)-phénol) ; le 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; le 3-(4-méthylbenzylidène)camphre ; le 3-benzylidènecamphre ; le salicylate d'éthylhexyle ; l'acide téréphtalidènedicamphosulfonique ; le 2-cyano-3,3-diphénylacrylate de 2-éthylhexyle ; l'ester 2-éthylhexylique de l'acide 4-(diméthylamino)-benzoïque ; l'ester amylique de l'acide 4-(diméthylamino)benzoïque ; l'ester di(2-éthylhexylique) de l'acide 4-méthoxybenzalmalonique ; l'ester 2-éthylhexylique de l'acide 4-méthoxycinnamique ; l'ester isoamylique de l'acide 4-méthoxycinnamique ; la 2-hydroxy-4-méthoxybenzophénone ; la 2-hydroxy-4-méthoxy-4'-méthylbenzophénone ; la 2,2'-dihydroxy-4-méthoxybenzophénone ; le salicylate d'homomenthyle ; le 2-hydroxybenzoate de 2-éthylhexyle ; le diméthicodiéthylbenzalmalonate ; un copolymère de 3-(4-(2,2-bis-éthoxycarbonylvinyl)-phénoxy)propényl)-méthoxysiloxane/diméthylsiloxane ; l'ester d'hexyle de l'acide 2-(4'-diéthylamino-2'-hydoxybenzoyl)-benzoïque ; la dioctylbutylamidotriazone (INCI : Diethylhexyl-Butamidotriazone) ; la 2,4-bis-[5-1(diméthylpropyl)benzoxazol-2-yl-(4-phényl)-imino]-6-(2-éthylhexyl)-imino-1,3,5-triazine (CAS n° 288254-16-0) ; la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) ; le triester de 2-éthylhexyle de l'acide 4,4',4"-(1,3,5-triazin-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; la 2,4,6-tribiphényl-4-yl-1,3,5-triazine ; la mérocyanine ; le dioxyde de titane ; l'oxyde de zinc.

10. Préparation cosmétique selon l'une quelconque des revendications précédentes, **caractérisée en ce que** la préparation contient un ou plusieurs composés choisis dans le groupe de composés formé par l'éthylhexylglycérine, le caprate de polyglycéryl-2, le propylèneglycol, le butylèneglycol, le 2-méthylpropane-1,3-diol, le 1,2-pentanediol, le 1,2-hexanediol, le 1,2-octanediol et/ou le 1,2-décanediol.

11. Préparation cosmétique selon l'une quelconque des revendications précédentes, **caractérisée en ce que** la préparation contient du phénoxyéthanol et/ou du méthylparabène.

12. Préparation cosmétique selon l'une quelconque des revendications précédentes, **caractérisée en ce que** la préparation contient un ou plusieurs principes actifs choisis dans le groupe de composés formé par un extrait de magnolias, l'acide alpha-lipoïque, l'acide folique, le phytoène, la D-biotine, la coenzyme Q10, l'alpha-glucosylrutine, la carnitine, la carnosine, le polydocanol, des isoflavonoïdes naturels et/ou synthétiques, des flavonoïdes, la créatine, la créatinine, la taurine, la β-alanine, l'acétate de tocophéryle, le panthénol, le magnolol, l'honokiol, l'urée, l'acide hyaluronique, la dihydroxyacétone, l'acide 8-hexadécène-1,16-dicarboxylique, l'acide glycyrrhétinique, des glucosylglycérides et/ou la licochalcone A.

13. Préparation cosmétique selon l'une quelconque des revendications précédentes, **caractérisée en ce que** la préparation est exempte de propylparabène et de butylparabène, de 3-iodo-2-propynylbutylcarbamate, de 3-(4-méthylbenzylidène)-camphre et de 2-hydroxy-4-méthoxybenzophénone (oxybenzone).

14. Préparation cosmétique selon l'une quelconque des revendications précédentes, **caractérisée en ce que** la préparation contient un benzoate d'alkyle en C12 à C15, une cyclométhicone, de l'octyldodécanol, des triglycérides d'acide caprylique/caprique, du dicaprylate/dicaprate de butylèneglycol, du benzoate de phénéthyle, des cocoglycérides, de l'adipate de di-n-butyle et/ou de l'adipate de diisopropyle.

15. Procédé pour la facilitation de la lessivabilité de préparations cosmétiques contenant du 4-(tert-butyl)-4'-méthoxydibenzoylméthane sur des textiles, **caractérisé en ce qu'**une gomme de cellulose et au moins un additif choisi dans le groupe de composés formé par l'iminodisuccinate, la 1-hydroxy-4-méthyl-6-(2,4,4-triméthylpentyl)-2(1H)-pyridone (piroctone) et l'acide diéthylènetriaminepenta(méthylènephosphonique)/ DTPMP sont ajoutés au produit cosmétique, des procédés avec les formulations
| | % en poids | % en poids | % en poids |
|---|---|---|---|
| Carboxyméthylcellulose | | 0,75 | 0,5 |
| Gomme de cellulose | 1,0 | 0,75 | 0,75 |
| EDTA trisodique | 0,20 | 0,20 | 0,50 |
| Iminodisuccinate tétrasodique | 0,75 | 0,50 | |
| acide diéthylènetriaminepenta(méthylènephosphonique) | | 0,50 | 0,50 |
| Butylméthoxydibenzoylméthane | 5,00 | 4,00 | 3,00 |
| Bis-éthylhexyloxyphénol-méthoxyphényltriazine | 3,50 | 3,50 | 0,50 |
| Diéthylaminohydroxybenzoylbenzoate d'hexyle | 0,50 | 1,00 | |
| Acide phénylbenzimidazolsulfonique | | 1,00 | 1,00 |
| Salicylate d'éthylhexyle | 4,50 | 5,00 | 5,00 |
| Dioxyde de titane | 3,00 | 2,00 | |
| Triméthoxycaprylylsilane | 0,20 | 0,20 | |
| Octocrylène | 10,00 | 10,00 | 10,00 |
| Homosalate | 4,50 | 10,00 | 10,00 |
| Alcool cétéarylique | | 1,00 | 0,50 |
| Gomme xanthane | 0,40 | 0,40 | 0,40 |
| Polymère croisé acrylates/acrylate d'alkyle en C10 à C30 | 0,30 | 0,05 | 0,20 |
| Alcool dénaturé | 5,00 | 4,00 | 6,00 |
| Méthylparabène | 0,30 | 0,30 | 0,30 |
| Phénoxyéthanol | 0,60 | 0,60 | 0,60 |
| Acide citrique | 0,30 | | |
| Citrate de sodium | 0,10 | | |
| Hydroxyde de sodium | 0,20 | 0,40 | 0,50 |
| Glycérine | 3,00 | 9,00 | 3,00 |
| Parfum | 0,50 | 0,40 | 0,60 |
| Copolymère VP/hexadécène | 0,50 | 0,50 | |
| Diméthylsilylate de silice | | 0,50 | 0,50 |
| Cétéarylsulfate de sodium | | 0,15 | |
| Stéarate de glycéryle SE | | 1,00 | |
| Stéarate citrate de glycéryle | 2,00 | | |
| Stéaroylglutamate de sodium | | | 0,40 |
| Stéarate de glycéryle | | | 1,00 |
| Coco-glycérides hydrogénés | 1,00 | | 1,00 |
| Benzoate d'alkyle en C12 à C15 | 5,00 | | |
| Myristate de myristyle | 1,00 | 1,00 | |
| Alcool stéarylique | 0,50 | | |
| Hydroxystéaroylstéarate d'alkyle en C18 à C38 | | 0,50 | |
| Stéarate d'isopropyle | 2,00 | | |
| Dicaprylate/dicaprate de butylèneglycol | 5,00 | | |
| Eau | qsp 100 | qsp 100 | qsp 100 |
étant exclus du domaine de protection.

16. Utilisation de gomme de cellulose et d'au moins un additif choisi dans le groupe de composés formé par l'iminodisuccinate, la 1-hydroxy-4-méthyl-6-(2,4,4-triméthylpentyl)-2(1H)-pyridone (piroctone) et l'acide diéthylènetriaminepenta(méthylènephosphonique)/ DTPMP dans des préparations cosmétiques contenant du 4-(tert-butyl)-4'-méthoxydibenzoylméthane pour la facilitation de la lessivabilité des filtres de protection contre les UV sur des textiles contaminés par les préparations, les formulations
| | % en poids | % en poids | % en poids |
|---|---|---|---|
| Carboxyméthylcellulose | | 0,75 | 0,5 |
| Gomme de cellulose | 1,0 | 0,75 | 0,75 |
| EDTA trisodique | 0,20 | 0,20 | 0,50 |
| Iminodisuccinate tétrasodique | 0,75 | 0,50 | |
| acide diéthylènetriaminepenta(méthylènephosphonique) | | 0,50 | 0,50 |
| Butylméthoxydibenzoylméthane | 5,00 | 4,00 | 3,00 |
| Bis-éthylhexyloxyphénol-méthoxyphényltriazine | 3,50 | 3,50 | 0,50 |
| Diéthylaminohydroxybenzoylbenzoate d'hexyle | 0,50 | 1,00 | |
| Acide phénylbenzimidazolsulfonique | | 1,00 | 1,00 |
| Salicylate d'éthylhexyle | 4,50 | 5,00 | 5,00 |
| Dioxyde de titane | 3,00 | 2,00 | |
| Triméthoxycaprylylsilane | 0,20 | 0,20 | |
| Octocrylène | 10,00 | 10,00 | 10,00 |
| Homosalate | 4,50 | 10,00 | 10,00 |
| Alcool cétéarylique | | 1,00 | 0,50 |
| Gomme xanthane | 0,40 | 0,40 | 0,40 |
| Polymère croisé acrylates/acrylate d'alkyle en C10 à C30 | 0,30 | 0,05 | 0,20 |
| Alcool dénaturé | 5,00 | 4,00 | 6,00 |
| Méthylparabène | 0,30 | 0,30 | 0,30 |
| Phénoxyéthanol | 0,60 | 0,60 | 0,60 |
| Acide citrique | 0,30 | | |
| Citrate de sodium | 0,10 | | |
| Hydroxyde de sodium | 0,20 | 0,40 | 0,50 |
| Glycérine | 3,00 | 9,00 | 3,00 |
| Parfum | 0,50 | 0,40 | 0,60 |
| Copolymère VP/hexadécène | 0,50 | 0,50 | |
| Diméthylsilylate de silice | | 0,50 | 0,50 |
| Cétéarylsulfate de sodium | | 0,15 | |
| Stéarate de glycéryle SE | | 1,00 | |
| Stéarate citrate de glycéryle | 2,00 | | |
| Stéaroylglutamate de sodium | | | 0,40 |
| Stéarate de glycéryle | | | 1,00 |
| Coco-glycérides hydrogénés | 1,00 | | 1,00 |
| Benzoate d'alkyle en C12 à C15 | 5,00 | | |
| Myristate de myristyle | 1,00 | 1,00 | |
| Alcool stéarylique | 0,50 | | |
| Hydroxystéaroylstéarate d'alkyle en C18 à C38 | | 0,50 | |
| Stéarate d'isopropyle | 2,00 | | |
| Dicaprylate/dicaprate de butylèneglycol | 5,00 | | |
| Eau | qsp 100 | qsp 100 | qsp 100 |
étant exclues du domaine de protection.
